# EUROPEAN PATENT APPLICATION

(11) **EP 4 129 154 A1**
(43) Date of publication of application: **08.02.2023**
(21) Application number: 21778796.9
(22) Date of filing: 25.03.2021
(51) Int. Cl.: A61B 3/028

(54) **OPTOMETRY SYSTEM, OPTOMETRY CONTROLLER, AND OPTOMETRY PROGRAM**

(30) Priority: 31.03.2020 JP 2020063522; 31.03.2020 JP 2020063523
(71) Applicant: Nidek Co., Ltd., Gamagori-shi, Aichi, 443-0038 (JP)
(72) Inventor: TERABE Hirohisa, Gamagori-shi Aichi 443-0038 (JP); HORINO Taeko, Gamagori-shi Aichi 443-0038 (JP)
(74) Representative: Hoefer & Partner Patentanwälte mbB
(86) International application number: PCT/JP2021/012743
(87) International publication number: WO 2021/200605

(57) **Abstract**

This optometry system is for subjectively measuring optical characteristics of an eye being examined, and is provided with: an optotype presenting unit for emitting optotype light flux toward the eye being examined; a correction means for changing an optical characteristic of the optotype light flux; an operation means for operating the optotype presenting unit and/or the correction means; a control means for controlling the optotype presenting unit and/or the correction means in response to an operation of the operation means; an acquisition means for acquiring a data log indicating a state of the optometry system when the control means controls the optotype presenting unit and/or the correction means; and an output means for outputting the data log. This makes it possible to accurately perform a subjective test even when an examiner and a subject are remote from each other.

## Description

### TECHNICAL FIELD

The present disclosure relates to an optometry system for subjectively measuring an optical characteristic of a subject eye, an optometry controller, and an optometry program.

### BACKGROUND ART

A subjective optometry device, in which an eye refractive power measurement unit is arranged in front of a subject eye, a visual target through an optical element (for example, a spherical lens, a cylindrical lens, and the like) is presented to the subject eye, and accordingly an eye refractive power or the like can be measured, is known (refer to Patent Literature 1).

### CITATION LIST

### PATENT LITERATURE

Patent Literature 1: JP2004-229769A

### SUMMARY OF INVENTION

In recent years, for various reasons, there has been a demand for a mechanism that makes it easy to perform a subjective examination, and the inventors have studied remote control of subjective examination. In some cases, an operator (assistant) who is unfamiliar with the examination proceeds with the subjective examination while receiving assistance from an examiner who is familiar with the examination.

As the first problem, it is considered that, when the examiner and the operator are apart from each other due to remote control of the subjective examination, it is difficult for the examiner to understand how the operator has proceeded with the examination, and it is difficult to accurately perform a subjective examination.

As the second problem, it is considered that, when the examiner and the operator are apart from each other due to remote control of the subjective examination, the examiner cannot give appropriate instructions to the operator, the operator cannot confirm the quality of the examination or the like with the examiner, and thus it is difficult to accurately perform a subjective examination.

In view of the conventional technology described above, the technical object of the present disclosure is to provide an optometry system, an optometry controller, and an optometry program capable of accurately performing a subjective examination even when the examiner and the examinee are apart from each other.

According to a first aspect of the present disclosure, there is provided an optometry system for subjectively measuring an optical characteristic of a subject eye, the optometry system including: a visual target presenting means that emits a target light flux toward the subject eye; a correction means that changes an optical characteristic of the target light flux; an operation means for operating at least one of the visual target presenting means and the correction means; a control means that controls at least one of the visual target presenting means and the correction means in response to an operation with respect to the operation means; an acquisition means that acquires a data log indicating a state of the optometry system in a case where the control means controls at least one of the visual target presenting means and the correction means; and an output means that outputs the data log.

According to a second aspect of the present disclosure, there is provided an optometry system for subjectively measuring an optical characteristic of a subject eye, the optometry system including: a visual target presenting means that emits a target light flux toward the subject eye; a correction means that changes an optical characteristic of the target light flux; a first operation means for operating at least one of the visual target presenting means and the correction means; a second operation means different from the first operation means; and a display control means that causes a display means of the first operation means to display a first instruction screen for transmitting, to the second operation means, a first trigger signal for controlling an action of the second operation means, in which the first trigger signal is transmitted from the first operation means to the second operation means in response to an operation with respect to the first instruction screen in the first operation means, and the second operation means outputs first instruction information for assisting an action of the optometry system, based on the first trigger signal transmitted from the first operation means.

According to a third aspect of the present disclosure, there is provided an optometry controller that operates an optometry system for subjectively measuring an optical characteristic of a subject eye, the optometry system including a visual target presenting means that emits a target light flux toward the subject eye, and a correction means that changes an optical characteristic of the target light flux, the optometry controller including: an operation means for operating at least one of the visual target presenting means and the correction means; a control means that controls at least one of the visual target presenting means and the correction means in response to an operation with respect to the operation means; an acquisition means that acquires a data log indicating a state of the optometry system in a case where the control means controls at least one of the visual target presenting means and the correction means; and an output means that outputs the data log.

According to a fourth aspect of the present disclosure, there is provided an optometry program used in an optometry system for subjectively measuring an optical characteristic of a subject eye, the optometry system including a visual target presenting means that emits a target light flux toward the subject eye, a correction means that changes an optical characteristic of the target light flux, an operation means for operating at least one of the visual target presenting means and the correction means, and a control unit that controls an action of the optometry system, the optometry program being executed by the control unit to cause the optometry system to perform: a control step of controlling at least one of the visual target presenting means and the correction means in response to an operation with respect to the operation means; an acquisition step of acquiring a data log indicating a state of the optometry system in a case where the control means controls at least one of the visual target presenting means and the correction means; and an output step of outputting the data log.

According to a fifth aspect of the present disclosure, there is provided an optometry program used in an optometry system for subjectively measuring an optical characteristic of a subject eye, the optometry system including a visual target presenting means that emits a target light flux toward the subject eye, a correction means that changes an optical characteristic of the target light flux, a first operation means for operating at least one of the visual target presenting means and the correction means, a second operation means different from the first operation means, a display control means that causes a display means of the first operation means to display a first instruction screen for transmitting, to the second operation means, a first trigger signal for controlling an action of the second operation means, and a control unit that controls an action of the optometry system, the optometry program being executed by the control unit to cause the optometry system to perform: a transmission step of transmitting the first trigger signal from the first operation means to the second operation means in response to an operation with respect to the first instruction screen in the first operation means; and an output step of causing the second operation means to output first instruction information for assisting an action of the optometry system, based on the first trigger signal transmitted from the first operation means.

### BRIEF DESCRIPTION OF DRAWINGS

[FIG. 1] FIG. 1 is an example of an optometry system.
[FIG. 2] FIG. 2 is an example of use of the optometry system.
[FIG. 3] FIG. 3 is a schematic view of an optometry device.
[FIG. 4] FIG. 4 is a schematic view of a light projecting optical system.
[FIG. 5] FIG. 5 is a schematic view of an eye refractive power measurement unit.
[FIG. 6] FIG. 6 is a schematic view of a corneal position targeting unit.
[FIG. 7A] FIG. 7A is an example of a first operation screen, a first instruction screen, and a data log screen of a monitor included in an examiner controller.
[FIG. 7B] FIG. 7B is an example of the data log screen of the monitor included in the examiner controller.
[FIG. 8] FIG. 8 is an example of the monitor included in an assistant controller.
[FIG. 9] FIG. 9 is a view showing a control system of the optometry system.

### DESCRIPTION OF EMBODIMENTS

### <Overview>

An overview of embodiments in the present disclosure will be described. The items classified by <> below can be used independently or in association with each other.

An optometry system (for example, an optometry system 1) according to the present embodiment is an optometry system for subjectively measuring an optical characteristic of a subject eye. Examples of the optical characteristic of the subject eye include at least one of eye refractive power (for example, spherical power, cylindrical power, astigmatism axis angle, prism amount, and the like), a binocular vision function (for example, prism amount, stereoscopic vision function, and the like), contrast sensitivity, and the like of the subject eye.

The optometry system may include an optometry device (for example, an optometry device 100). For example, the optometry device may include at least a forehead pad (for example, a forehead pad 121) and a correction means that will be described later. The forehead pad may be provided in the correction means, and the subject eye is arranged at a predetermined position with respect to the correction means by bringing the head portion of the examinee into contact with the forehead pad.

The optometry system may include an optometry controller. The optometry controller may be configured by a first operation means (for example, a controller 200). Further, the optometry controller may be configured by the first operation means and a second operation means (for example, a controller 400). In this case, one of the first operation means and the second operation means may be an examiner operation means operated by the examiner, and the other one may be an assistant operation means operated by the assistant who assists the operation of the examiner or the examinee.

The examination device and the operation means (at least one of the first operation means and the second operation means) may include a communication means. For example, the examination device and the operation means may be directly connected and communicate with each other by using the communication means. Further, for example, the examination device and the operation means may be indirectly connected and communicate with each other by using the communication means. It is needless to say that, in a case where the operation means includes the first operation means and the second operation means, each operation means may be directly or indirectly connected and communicate with each other by using the communication means. The indirect connection between the optometry device and the operation means, and the indirect connection between the first operation means and the second operation means may be via a shared server (for example, a shared server 300).

### <Visual target presenting means>

In the present embodiment, the optometry system includes a visual target presenting means. The visual target presenting means emits a target light flux toward the subject eye.

The visual target presenting means may be a display (for example, a display 161). In this case, any one of LCOS, LCD, organic EL, and the like can be used. Further, the visual target presenting means may be a light source and a digital micromirror device (DMD). Further, the visual target presenting means may be a light source and a visual target plate.

The target light flux from the visual target presenting means may be directly emitted toward the subject eye. Further, the target light flux from the visual target presenting means may be indirectly guided toward the subject eye via a light projecting optical system (for example, a light projecting optical system 160). The light projecting optical system may include at least one optical member for passing the target light flux. For example, the light projecting optical system may include a lens, a mirror, or the like.

For example, the optometry system may include the visual target presenting means as a visual target presentation device, in addition to the optometry device. Further, for example, the optometry system may include the visual target presenting means as a member that partially configures the optometry device. In this case, the optometry device may be provided in the visual target presenting means.

### <Correction means>

In the present embodiment, the optometry system includes a correction means. The correction means is arranged in front of the subject eye and changes an optical characteristic of the target light flux. For example, the optical characteristic of the target light flux may be at least one of spherical power, cylindrical power, astigmatism axis angle, prism amount, and the like of the target light flux. For example, the target light flux emitted from the visual target presenting means is guided to the subject eye via the correction means.

The correction means may include a correction optical system. The correction optical system may have any configuration as long as it is possible to change the optical characteristic of the target light flux.

As an example, the correction optical system may include an optical element (for example, an optical element 133) and control the optical element to change the optical characteristic of the target light flux. For example, the optical element may be at least one of a spherical lens, a cylindrical lens, a cross cylinder lens, a rotary prism, a wavefront modulation element, a varifocal lens, and the like. It is needless to say that the optical elements may differ from these.

Further, as an example, the correction optical system may optically change a presentation position (presentation distance) of the visual target with respect to the subject eye to change the optical characteristic of the target light flux. In this case, the visual target presenting means may be moved in the optical axis direction, or an optical element (for example, a spherical lens or the like) arranged in the optical path may be moved in the optical axis direction.

Further, for example, the correction optical system may arrange an optical element between the visual target presenting means and the optical member for guiding the target light flux from the light projecting optical system toward the subject eye, and control the optical element, to change the optical characteristic of the target light flux. In other words, the correction optical system may be a phantom lens refractometer (phantom correction optical system).

In the present embodiment, an eye refractive power measurement unit (for example, an eye refractive power measurement unit 120) that arranges an optical element in front of the subject eye is used as the correction means. For example, the eye refractive power measurement unit may include a varifocal lens as an optical element, and change the refractive power of the varifocal lens to correct the subject eye. Further, for example, the eye refractive power measurement unit may include a lens disk (for example, a lens disk 131) in which a plurality of optical elements are arranged on the same circumference and a driving means (for example, a driving unit 134) for rotating the lens disk, and may drive the driving means and switching the optical element to correct the subject eye. It is needless to say that the eye refractive power measurement unit may include a varifocal lens, a lens disk, and a driving means, and may control these to correct the subject eye.

### <Control means>

In the present embodiment, the optometry system includes a control means (for example, a control unit 170). The control means controls at least one of the visual target presenting means and the correction means in response to an operation with respect to the operation means (at least one of the first operation means and the second operation means).

The control means may perform control that changes the visual target to be displayed on the visual target presenting means, based on an operation signal input from the operation means. For example, control that changes a type of the visual target, a size of the visual target, a position of the visual target, and the like, may be performed. Further, the control means may perform control that changes the correction optical system, based on an operation signal input from the operation means. For example, control that changes the arrangement of optical elements or optical members may be performed.

### <Acquisition means>

In the present embodiment, the optometry system includes an acquisition means (for example, a control unit 170). The acquisition means acquires a data log indicating a state of the optometry system in a case where the control means controls the optometry system. For example, the acquisition means acquires a data log indicating the state of the optometry system in a case where the control means controls at least one of the visual target presenting means and the correction means. For example, the data log may be data with chronological order recorded by controlling the optometry system.

For example, the acquisition means may acquire a data log indicating a state of the visual target presenting means in a case where the control means controls the visual target presenting means. As an example, the data log may be a data log of visual target information that will be described later. Further, for example, the acquisition means may acquire a data log indicating a state of the correction means in a case where the control means controls the correction means. As an example, the data log may be a data log of correction information that will be described later. It is needless to say that the data log may be both the data log indicating the state of the visual target presenting means and the data log indicating the state of the correction means.

The data log indicating the state of the optometry system may include at least one of the visual target information (for example, visual target information 224) related to the visual target presented to the subject eye, and the correction information (for example, correction information 225) related to correction of the subject eye. By using these data logs, the examiner can easily determine whether or not the visual target presented to the subject eye is appropriate, or whether or not the correction state of the subject eye is appropriate.

For example, the data log of the visual target information may be a data log of at least one of the type of the visual target and the visual acuity value of the visual target. For example, by using such a data log, it is possible to read what type of examination the assistant performed on the subject eye. As an example, in a case where a point group visual target is presented, it is possible to read that a cross-cylinder test has been performed. In addition, for example, by using such a data log, it is possible to read changes in the type or the visual acuity value of the visual target, the number of times (frequency) of switching the type or the visual acuity value of the visual target, and the like.

For example, a data log of the correction information may be a data log of at least one of the target eye of the subjective examination on the subject eye, the adjustment item of which a correction power is adjusted with respect to the subject eye, and the correction power value by which the correction means corrects the subject eye. For example, by using such a data log, it is possible to read whether the left eye or the right eye has been corrected, the number of times (frequency) of switching the correction item, the number of times (frequency) of switching the correction power, changes in correction power value, and the like.

For example, the adjustment item is an item indicating which eye refractive power of the subject eye has been changed by controlling the optical element by the correction means. In other words, for example, the adjustment item is an item indicating which optical characteristic of the target light flux has been changed by controlling the optical element by the correction means. As an example, the adjustment item corresponds to one of the spherical power, the cylindrical power, the astigmatism axis angle, the prism, and the like.

It is needless to say that the data log indicating the state of the optometry system may include a data log of information other than the visual target information and the correction information. For example, a data log indicating the state of an optometry program may be included. In this case, the visual target information and the correction information may be associated with each examination item that the optometry program proceeds with. That is, the visual target information and the correction information for each examination item may be acquired. Further, for example, a data log indicating a progress of measurement with respect to the subject eye may be included. In this case, elapsed time required from a start to an end of measurement for each examination item may be acquired. As an example, such a log indicating the elapsed time may be used to determine whether the examinee is burdened by an extension of the measurement time, whether the assistant is familiar with measurement, whether the assistant has gradually become familiar with the measurement if the assistant is unfamiliar with measurement, or the like.

### <Extraction means>

In the present embodiment, the optometry system includes an extraction means (for example, a control unit 170). The extraction means extracts a specific data log from the data log indicating the state of the optometry system acquired by the acquisition means.

For example, the extraction means may extract a specific data log in units of data logs acquired each time the control means controls at least one of the visual target presenting means and the correction means. In other words, the extraction means may extract a specific data log in units of one operation in which the control means controls at least one of the visual target presenting means and the correction means.

As an example, in a case where the control means switches a state where the visual target is set to a predetermined visual acuity value (for example, 1.0) to a first visual acuity value (for example, 0.8), and further switches to a second visual acuity value (for example, 0.7), an operation by the control means becomes two operations. Further, as an example, in a case where the control means switches a state where the correction power value is set to a predetermined spherical power value (for example, -3.5 D), a cylindrical power value (for example, -1.0 D), and an astigmatism axis angle, to a first spherical power value (for example, -3.25 D), then switches to a second spherical power value (for example, -3.0 D), and further switches to a first cylindrical power value (for example, -0.5 D), the operation by the control means becomes two operations.

In a subjective examination, at least one of the visual target presenting means and the correction means is controlled by the control means. Therefore, in practice, the operation is a combination of changes in the type of the visual target, the visual acuity value of the visual target, the target eye, the adjustment item, the correction power values (the spherical power value, the cylindrical power value, and the astigmatism axis angle), and the like.

In this case, the specific data log may include a data log obtained at at least one operation before the timing of the predetermined operation. That is, the specific data log may be only the data log obtained at one operation before, or may be the range indicated by the data log from the log one operation before to the log before any operation.

For example, the extraction means may extract a specific data log in units of data logs that is acquired each time the control means controls at least one of the visual target presenting means and the correction means and is associated with the examination item of the subjective examination. In other words, the extraction means may extract a specific data log in units of one examination in which the control means controls at least one of the visual target presenting means and the correction means.

As an example, all of the data logs such as the type of the visual target, the visual acuity value of the visual target, the target eye, the adjustment item, and the correction power value that are switched by the control means during the spherical test in the subjective examination, may be extracted as specific data logs. In addition, all of the data logs such as the type of the visual target, the visual acuity value of the visual target, the target eye, the adjustment item, and the correction power value that are switched by the control means during the astigmatism test in the subjective examination, may be extracted as specific data logs. It is needless to say that each and every data log switched during an examination other than the spherical surface test or the astigmatism test, may be extracted as specific data logs.

In this case, the specific data log may include the data log obtained at at least one examination before after the timing of the predetermined examination. That is, the specific data log may be only the data log obtained at one examination before, or may be the range indicated by the data log from the log obtained at one examination before to the log before any examination.

In addition, in a case where the specific data log is the data log in the range from the log obtained at one operation before to any operation or from the log obtained at one examination before to any examination, the operator may designate the range in any manner, or a predetermined number of operations or examinations may be set in advance.

### <First operation means/second operation means>

In the present embodiment, the optometry system includes an operation means. The operation means is an operation means for operating at least one of the visual target presenting means and the correction means.

Any operation means may be used as the operation means as long as input is possible by the operator (for example, the examiner or the assistant). As an example, the operation means may be at least any user interface such as a switch, a touch panel, a mouse, a keyboard, or the like. In addition, in a case where the operation means is a touch panel, the operation means also serves as the display means.

The operation means can input an operation signal for operating the visual target presenting means. For example, the operation means can input an operation signal for changing at least one of the type of the visual target, the visual acuity value of the visual target, the presentation distance of the visual target, and the like, which are displayed by the visual target presenting means. Further, the operation means can input an operation signal for operating the correction means. For example, the operation means can input an operation signal for changing at least one of the correction power for correcting the subject eye, the arrangement of the optical elements, and the like. It is needless to say that the operation means may be configured to input an operation signal for operating a means other than the visual target presenting means and the correction means.

In the present embodiment, the optometry system includes a first operation means (for example, a controller 200) and a second operation means (for example, a controller 400).

For example, the first operation means may be an operation means for operating at least one of the visual target presenting means and the correction means, and the second operation means may be an operation means different from the first operation means. In this case, similarly to the first operation means, the second operation means may have a function as the operation means for operating at least one of the visual target presenting means and the correction means. In addition, in this case, unlike the first operation means, the second operation means may not have a function as the operation means for operating at least one of the visual target presenting means and the correction means.

Further, for example, the first operation means may be an operation means different from the second operation means, and the second operation means may be operation means for operating at least one of the visual target presenting means and the correction means. In this case, similarly to the second operation means, the first operation means may have a function as the operation means for operating at least one of the visual target presenting means and the correction means. In addition, in this case, unlike the second operation means, the first operation means may not have a function as the operation means for operating at least one of the visual target presenting means and the correction means.

In other words, only one of the first operation means and the second operation means may be capable of operating at least one of the visual target presenting means and the correction means. In addition, both the first operation means and the second operation means may be capable of operating at least one of the visual target presenting means and the correction means.

The first operation means has a display means (for example, a monitor 220). The display means is configured to display a first instruction screen (for example, a first instruction screen 222) for transmitting, to the second operation means, a first trigger signal for controlling an action of the second operation means.

For example, the trigger signal for controlling the action of the second operation means may be a signal that serves as a trigger for causing the second operation means to output first instruction information that will be described later. As an example, the second operation means may have a display means (for example, a monitor 420), and the trigger signal is a signal for causing the display means to display the first instruction information. Further, as an example, the second operation means may include a voice generation means (for example, a speaker), and the trigger signal is a signal for causing the voice generation means to generate the first instruction information. Further, as an example, the second operation means may include a printing means (for example, a printer), and the trigger signal is a signal for causing the printing means to print the first instruction information. It is needless to say that the trigger signal may be a signal for outputting the first instruction information in another output method different from these.

In the present embodiment, the first trigger signal is transmitted from the first operation means to the second operation means in response to an operation with respect to the first instruction screen in the first operation means. That is, a trigger signal for causing the second operation means to output the first instruction information that will be described later. For example, at least one sign may be displayed on the first instruction screen. The first trigger signal is associated with the sign. In addition, at least one of characters, symbols, figures, numbers, icons, and the like that represents the content of the first trigger signal, is used as the sign. In this case, the operation with respect to the first instruction screen may be a direct operation with respect to the first instruction screen. As an example, a touch panel may be operated using a finger, a touch pen, or the like. Further, in this case, the operation with respect to the first instruction screen may be an indirect operation with respect to the instruction screen. As an example, an operation using at least one of a mouse, a keyboard, a dial, a joystick, and the like may be performed. For example, in a case where the first instruction screen of the first operation means displays a sign in this manner, the first operation means can transmit the first trigger signal to the second operation means, based on the operation with respect to the sign.

Further, for example, the first instruction screen may include a writing area in which characters or the like can be written in any manner. In this case, handwritten information input with a finger or a touch pen may be converted into the first trigger signal. Further, for example, the first instruction screen may include a typing area in which characters or the like can be typed in any manner. In this case, character information input by a keyboard or the like may be converted into the first trigger signal. Further, for example, the instruction screen may include a voice display area capable of displaying any voice as characters. In this case, character information input by a microphone or the like may be converted into the first trigger signal. For example, in a case where the first instruction screen of the first operation means includes respective areas in this manner, the first operation means can transmit the first trigger signal to the second operation means, based on the operation with respect to the respective areas.

In the present embodiment, the second operation means outputs the first instruction information for assisting the action of the optometry system, based on the first trigger signal transmitted from the first operation means. That is, the second operation means controls the operation of the second operation means based on the first trigger signal transmitted from the first operation means to output first instruction information for assisting the action of the optometry system. As a result, even when the examiner is apart from the operator, the examiner can easily convey the contents of the suggestions or instructions to the assistant, and the subjective examination can be accurately performed.

For example, the first instruction information for assisting the action of the optometry system may include at least information for assisting measurement posture in a case where the examinee observes the visual target presented by the visual target presenting means. In this case, information for assisting eyelid-open state (for example, the squint state) of the subject eye may be included. In this case, information for assisting the position of the correction means with respect to the subject eye may also be included. As an example, the center positions of the pupils of the left eye and the right eye and the positions of the optical elements of the correction means corresponding to the left eye and the right eye, may be used. Further, as an example, the corneal apex position of the subject eye and the position of the optical element of the correction means, may be used. Further, as an example, the positions of the head portion of the examinee and the forehead pad may be used. For example, by confirming the first instruction information, the examiner can determine the quality of the measurement posture of the examinee, and can convey appropriate instructions to the operator.

Further, the instruction information for assisting the action of the optometry system may include at least information for assisting the procedure for operating at least one of the visual target presenting means and the correction means. In this case, information for assisting the procedure for operating the visual target presenting means may be included. As an example, at least one of the change in the type of the visual target to be presented by the visual target presenting means, the change in the visual acuity value of the visual target, and the like may be used. In this case, information for assisting the procedure for operating the correction means may also be included. As an example, at least one of the change in correction power, the change in adjustment item, and the like may be used. By confirming the first instruction information, the examiner can determine the quality of the operation procedure by the assistant, and can convey appropriate instructions to the operator.

It is needless to say that the first instruction information for assisting the action of the optometry system may include information different from the information for assisting the above-described measurement posture or operation procedure. For example, information such as at least one of re-execution of the operation, end of the operation, and the like may be included. Further, for example, information of requests (for example, inquiries, answers, and the like) from examiners or assistants may be included.

### <Setting means>

In the present embodiment, the optometry system includes a setting means (for example, a control unit 280). The setting means sets an arrangement of the signs on the first instruction screen in the first operation means. In other words, the setting means sets alignment order of the signs on the first instruction screen in the first operation means. Accordingly, the examiner can easily select necessary signs according to the examination items with respect to the subject eye, or the like.

For example, the arrangement of signs may be set manually. For example, the setting means may set the arrangement of the signs, based on a setting signal input by the operator operating the operation means. In this case, the setting means may store each sign in association with the display position of each sign on the instruction screen.

Further, for example, the arrangement of signs may be set automatically. For example, the setting means may set the arrangement of the signs, based on the detection results of detected frequency of use of the signs. Further, for example, the setting means may set the arrangement of the signs, based on the examination items of the subjective examination. For example, a predetermined sign may be set for each examination mode, based on switching of the examination mode by the operator, switching of the examination mode by programmed optometry, or the like.

### <Output means>

In the present embodiment, the optometry system includes an output means (for example, a control unit 280). The output means outputs a data log acquired by the acquisition means. For example, the output means may output all data logs acquired by the acquisition means. Further, for example, the output means may output a specific data log extracted by the extraction means. As a result, even when the examiner is apart from the operator, the examiner can easily confirm the suitability of the subjective examination by the operator.

For example, the output means may output the data log acquired by the acquisition means such that the state of the optometry system can be understood. That is, for example, the output means may output a data log analyzed such that the state of the visual target presenting means can be recognized. Further, for example, the output means may output a data log analyzed such that the state of the correction means can be recognized.

For example, the output means may be configured to output the data log acquired by the acquisition means to the outside. In this case, the output means may output the data log by at least one of display on the display means, generation of voice guide, storage in a memory or a server, printing on a printer or the like, transmission to an external device, and the like.

For example, the output means may switch an output from an output of a first data log indicating a part of the data log to an output including at least a second data log different from the first data log indicating a part of the data log, according to a switching signal for switching an output form by the output means. For example, a switching signal may be input by an operation or the like by the operator, and the output form of the data log may be switched accordingly.

For example, the output means may switch an output form from an output of the first data log to an output of the second data log. As an example, a switching signal may be input by at least one of a scrolling operation, a swiping operation, and the like by the operator, and the data log hidden outside the display screen of the display means may be displayed on the display screen of the display means. Further, for example, the output means may switch the output form from the output of the first data log to the output of the first data log and the second data log. As an example, a switching signal may be input by a tap operation or the like by the operator, and the data log folded on the display screen of the display means may be displayed on the display screen of the display means.

### <Display control means>

In the present embodiment, the optometry system includes a display control means (for example, a control unit 280). The display control means causes the display means of the first operation means to display the above-described first instruction screen for transmitting, to the second operation means, the first trigger signal for controlling the action of the second operation means.

The display control means may cause the display means of the first operation means to display a screen different from the first instruction screen. For example, an operation screen for transmitting an operation signal for controlling at least one of the visual target presenting means and the correction means may be displayed. Further, for example, a data log screen for displaying the data log acquired by the acquisition means may be displayed.

For example, the display control means may display a sign on the first instruction screen. In this case, the display control means may display all signs prepared in advance. Further, in this case, the display control means may display a predetermined sign among all of the signs prepared in advance. Further, for example, the display control means may rearrange these signs, based on the arrangement set by the setting means.

Further, the display control means may cause the display means to display the data log acquired by the acquisition means. In this case, the display control means may cause the display means included in the first operation means to display the data log acquired by the acquisition means. Further, in this case, the display control means may cause display means different from the display means included in the first operation means to display the data log acquired by the acquisition means.

In addition, in the present embodiment, the second display means may include a display means and be configured to be capable of transmitting the trigger signal similarly to the first display means. More specifically, a configuration may be adopted in which the display control means cause the display means of the second operation means to display a second instruction screen for transmitting, to the first operation means, a second trigger signal for controlling the action of the first operation means, the second trigger signal is transmitted from the second operation means to the first operation means in response to an operation with respect to the second instruction screen in the second operation means, the first operation means outputs the second instruction information for assisting the action of the optometry system, based on the second trigger signal transmitted from the second operation means, and the first operation means and the second operation means can communicate with each other.

For example, accordingly, the examiner and assistant can send instruction information to each other. More specifically, even when the examiner is apart from the assistant, the examiner can easily give appropriate instructions to the assistant using the instruction information. In addition, even when the assistant is apart from the examiner, the assistant can easily ask the examiner about the quality of the examination, or the like. As a result, the examiner and the assistant can easily convey instruction contents or suggestion contents to each other, and can accurately proceed with the subjective examination while confirming each other.

The present disclosure is not limited to the optometry system described in the present embodiment. For example, terminal control software (program) that performs the functions of the above-described embodiment is supplied to the optometry system via a network or various storage media, and the control device (for example, CPU or the like) of the optometry system can read and execute the program.

### <Example>

An example of the optometry system related to the present embodiment will be described with reference to the drawings.

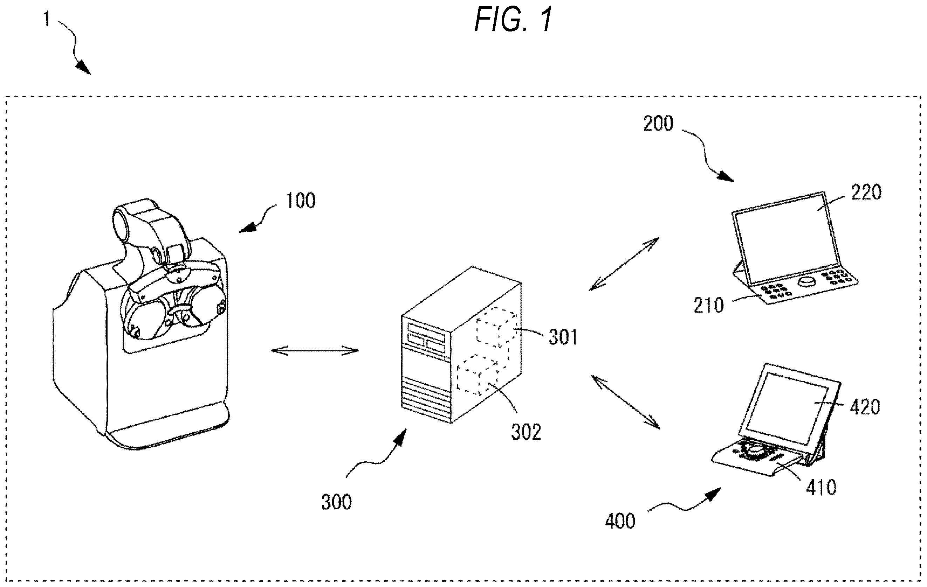

FIG. 1 is an example of an optometry system 1. The optometry system 1 includes a subjective optometry device 100 (hereinafter referred to as an optometry device 100), an optometry controller 200 for an examiner (hereinafter referred to as a controller 200), an optometry controller 400 for an assistant (hereinafter referred to as a controller 400), and the like. The optometry device 100, the controller 200, and the controller 400 include a communication unit that is not shown and are connected to each other via a wired or wireless network.

For example, in the optometry system 1, the optometry device 100, the controller 200, and the controller 400 are connected via a shared server 300. The shared server 300 receives a control command from the optometry device 100 and controls at least one of the controller 200 and the controller 400. Further, the shared server 300 receives a control command from at least one of the controller 200 and the controller 400 and controls the optometry device 100. Further, the shared server 300 receives a control command from the controller 200 and controls the controller 400. Further, the shared server 300 receives a control command from the controller 400 and controls the controller 200.

The shared server 300 is not necessarily an essential component, and at least one of the controller 200 and the controller 400 may receive a control command from the optometry device 100. Further, the optometry device 100 may receive a control command from at least one of the controller 200 and the controller 400. Further, the controller 400 may receive a control command from the controller 200. Further, the controller 200 may receive a control command from the controller 400.

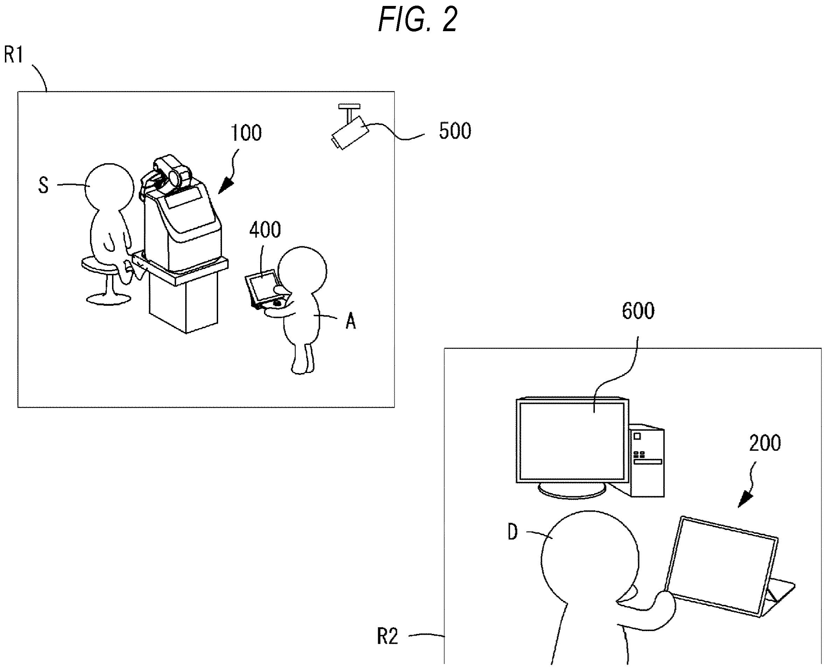

FIG. 2 is an example of use of the optometry system 1. In FIG. 2, a case where the optometry device 100 and the controller 400 are installed in an examination room R1 where an examinee S, an examiner D, and an assistant A who assists the examinee S are present, and the controller 200 is arranged in an observation room R2 where the examiner D is present is taken as an example. That is, a case where the assistant A operates the optometry device 100 with the controller 400 provided for the assistant, and the examiner D remotely operates the optometry device 100 with the controller 200 provided for the examiner, is taken as an example.

Operations that can be controlled using the controller 200 and operations that can be controlled using the controller 400 may be assigned in advance to each operation of the optometry device 100. In addition, a photographing camera 500 for photographing the inside of the examination room R1 may be installed in the examination room R1. Further, a monitor 600 that displays a video of the photographing camera 500 may be installed in the observation room R2.

### <Optometry device>

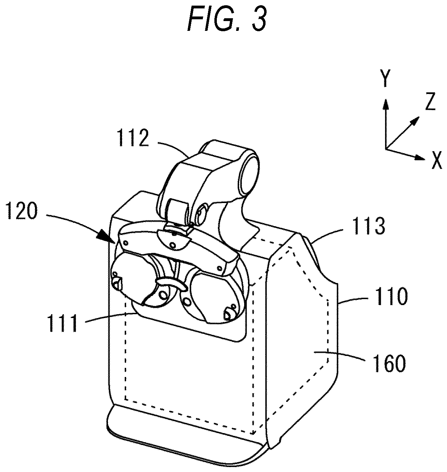

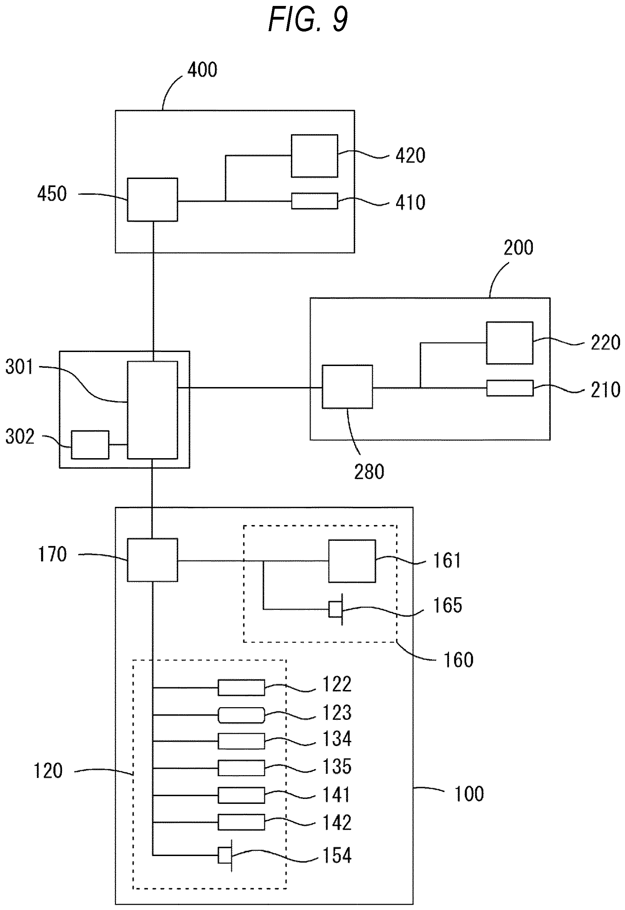

FIG. 3 is a schematic view of the optometry device 100. For example, the optometry device 100 includes a housing 110, a presentation window 111, a holding arm 112, an observation window 113, an eye refractive power measurement unit 120 (hereinafter referred to as a measurement unit 120), a control unit 170 (refer to FIG. 9), and the like.

The presentation window 111 is provided in the front surface of the housing 110. The light projecting optical system 160 that will be described later is accommodated inside the housing 110. The observation window 113 is provided in the rear surface of the housing 110.

The presentation window 111 is a window for presenting the visual target to the subject eye. The presentation window 111 transmits the target light flux in the light projecting optical system 160 and projects the target light flux through the presentation window 111 onto the subject eye.

The holding arm 112 holds the measurement unit 120. The holding arm 112 holds the measurement unit 120 to be arranged at a predetermined position. For example, in the present example, the measurement unit 120 is held to be arranged at at least one of the measurement position where the measurement unit 120 is lowered on the front surface of the housing 2 or the standby position where the measurement unit 120 is raised above the housing 2. Switching between the measurement position and the retracted position by the holding arm 112 is performed by control of a driving unit that is not shown.

The observation window 113 is a window for confirming the positions of the center of the pupil of a subject eye E and an optometry window 132 that will be described later. The observation window 113 is arranged at a position where the pupil of the subject eye E can be observed. Further, the observation window 113 is arranged out of the optical path through which the target light flux passes in the light projecting optical system 160 that will be described later. In the present example, the observation window 113 is arranged above the optical path of the target light flux to be out of the optical path. As a result, the visual target displayed on the display 161 is presented to the subject eye E without missing.

For example, the assistant A (assistant eye AE) can understand the positional relationship between the subject eye E and the measurement unit 120 by looking into the observation window 113 and observing the positions of the subject eye E and the optometry window 132, through the presentation window 111, from the outside of the housing 110.

### <Light projecting optical system>

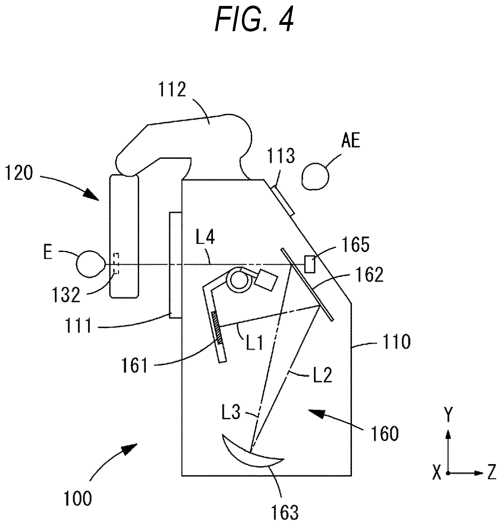

FIG. 4 is a schematic view of the light projecting optical system 160. For example, the light projecting optical system 160 includes a display 161, a plane mirror 162, a concave mirror 163, a half mirror 164, an imaging element 165, and the like.

The visual target (for example, fixation target, examination visual target, and the like) is displayed on the display 161. The target light flux emitted from the display 161 is guided to the subject eye E. For example, the target light flux emitted from the display 161 is guided to the subject eye E via the measurement unit 120 arranged in front of the subject eye E. The display of the display 161 is controlled by the control unit 170 that will be described later.

The plane mirror 162 reflects the target light flux from the display 161 and guides the target light flux to the concave mirror 163. Instead of the plane mirror 162, a prism, a beam splitter, a half mirror, or the like may be used to guide the target light flux from the display 161 to the concave mirror 163.

The concave mirror 163 reflects the target light flux from the display 161 and guides the target light flux to the half mirror 164. For example, focal length of the concave mirror 163 is designed such that the optical distance from the display 161 to the subject eye E is 5 m. Instead of the concave mirror 163, an aspherical mirror, a free-form surface mirror, a lens, or the like may be used to guide the target light flux from the display 161 to the half mirror 164.

The half mirror 164 reflects the target light flux from the display 161 and guides the target light flux to the subject eye E. Instead of the half mirror 164, a prism, a beam splitter, a plane mirror, or the like may be used to guide the target light flux from the display 161 to the subject eye E.

The imaging element 165 is arranged in the transmission direction of the half mirror 164. The imaging element 165 captures the subject eye E, the measurement unit 120, and the subject eye E from the front. For example, a captured image (first image) captured by the imaging element 165 may be transmitted to the controller 200 via the shared server 300 and may be displayed on the monitor 220 of the controller 200. For example, the examiner D can understand the positional relationship between the subject eye E and the measurement unit 120 by observing the positions of the subject eye E and the optometry window 132, using the first image. Imaging by the imaging element 165 is controlled by the control unit 170.

For example, during a subjective examination of the subject eye E, the target light flux emitted from the display 161 passes through an optical axis L1 and is reflected by the plane mirror 162, passes through an optical axis L2 and is reflected by the concave mirror 163, passes through an optical axis L3 and is reflected by the half mirror 164, and passes through an optical axis L4 and is projected onto the subject eye E.

### <Eye refractive power measurement unit>

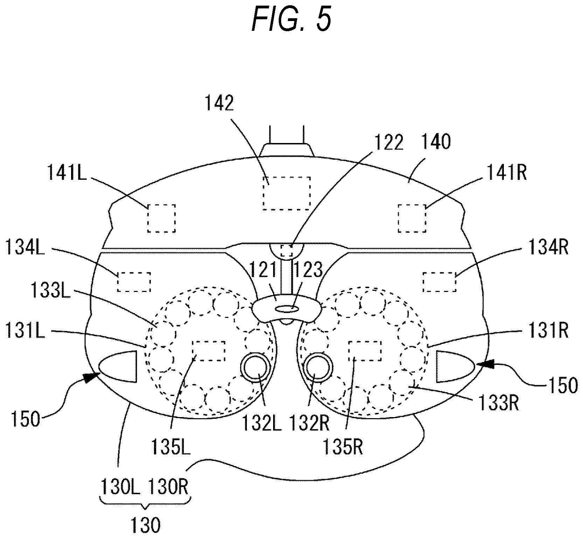

FIG. 5 is a schematic view of the measurement unit 120. The measurement unit 120 includes the forehead pad 121, a lens unit 130, a moving unit 140, a corneal position targeting unit 150 (hereinafter referred to as a targeting unit 150), and the like.

The head portion of the examinee is brought into contact with the forehead pad 121. The position of the forehead pad 121 in the front-rear direction (Z direction) is adjusted by a driving unit 122. Therefore, the subject eye E is kept at a predetermined distance from the optometry window 132 that will be described later. For example, the position of the forehead pad 121 can be appropriately changed by outputting an operation signal from the controller 200 and controlling the driving unit 122. Further, for example, the position of the forehead pad 121 can be appropriately changed by outputting an operation signal from the controller 400 and controlling the driving unit 122. For example, the driving unit 122 may be configured by a motor, a solenoid, or the like.

The forehead pad 121 is provided with a detector 123. The detector 123 detects contact between the head portion of the examinee and the forehead pad 121. For example, the detector 123 may be an optical sensor, a pressure sensor, a load sensor, or the like.

The lens unit 130 has a pair of leftlens unit 130L and right lens unit 130R. The lens unit 130 includes the lens disk 131 (a left lens disk 131L and a right lens disk 131R), the optometry window 132 (a left optometry window 132L and a right optometry window 132R), and the like.

A plurality of optical elements 133 (a left optical element 133L and a right optical element 133R) are arranged on the same circumference of the lens disk 131. For example, the optical element 133 may be a spherical lens, a cylindrical lens, a prism lens, or the like. For details of the lens disk 131 and the optical element 133, refer to, for example, JP2007-068574A.

The lens disk 131 is rotated by the driving unit 134 (a left driving unit 134L and a right driving unit 134R). For example, the rotation angle of the lens disk 131 can be appropriately changed by outputting an operation signal from the controller 200 and controlling the driving unit 134. Further, the optical element 133 is rotated by a driving unit 135 (a left driving unit 135L and a right driving unit 135R). For example, the rotation angle of the optical element 133 can be appropriately changed by outputting an operation signal from the controller 200 and controlling the driving unit 135. For example, the driving unit 134 and the driving unit 135 may be configured by a motor, a solenoid, or the like.

The optical elements 133 are switched and arranged in the optometry window 132 as the lens disk 131 rotates. Therefore, the optical element 133 desired by the examiner is arranged at a desired rotation angle in front of the subject eye E.

The moving unit 140 moves the lens unit 130 in the left-right direction (X direction) by a driving unit 141 (a left driving unit 141L and a right driving unit 141R). For example, the distance between each lens unit is adjusted by moving the left lens unit 130L by the left driving unit 141L and moving the right lens unit 130R by the right driving unit 141R. For example, the movement of the lens unit 130 can be appropriately changed by outputting an operation signal from the controller 200 and controlling the driving unit 141. For example, the driving unit 141 may be configured by a motor, a slide mechanism, and the like. Accordingly, the interval between the optometry windows 132 can be adjusted according to a pupillary distance PD of the subject eye E.

Further, the moving unit 140 rotates the lens unit 130 in the convergence direction by a driving unit 142. For example, by rotating both the left lens unit 130L and the right lens unit 130R by the driving unit 142, the convergence angle (inward angle) of each lens unit is adjusted. For example, the rotation of the lens unit 130 is appropriately changed by controlling the driving unit 142 according to the pupillary distance PD of the subject eye E, the distance from the subject eye E to the visual target (that is, the presentation distance for presenting the visual target to the subject eye E), and the like. For example, the driving unit 141 may be configured by a motor, a convergence mechanism, and the like.

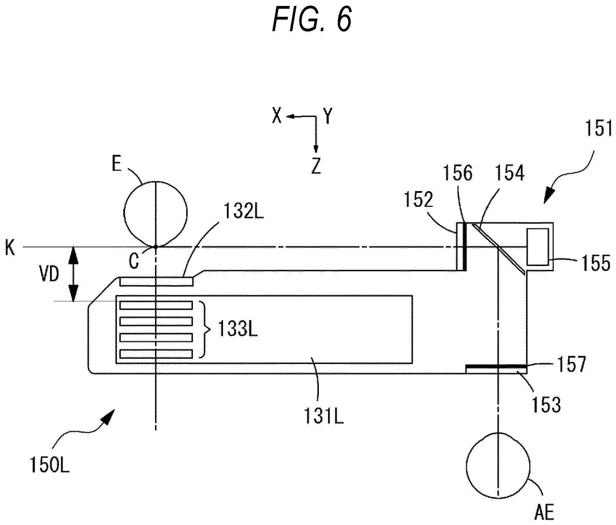

FIG. 6 is a schematic view of the targeting unit 150. In FIG. 6, a left targeting unit 150L provided in the left lens unit 130L is shown, and the right targeting unit 150R provided in the right lens unit 130R will be omitted since a right targeting unit 150R has a symmetrical configuration. The left targeting unit 150L include a targeting optical system 151. The targeting optical system 151 includes a first observation window 152, a second observation window 153, a half mirror 154, an imaging element 155, and the like.

The first observation window 152 and the second observation window 153 are windows for confirming the distance between the corneal apex position of the subject eye E and the reference position in a case where the lens is worn. The first observation window 152 is arranged in one reflection direction on the half mirror 154. A targeting scale plate 156 (refer to FIG. 6) is provided in the first observation window 152. The second observation window 153 is arranged in the other reflection direction on the half mirror 154. A reticle plate 157 (refer to FIG. 6) is provided in the second observation window 153. For the detailed configuration of the targeting scale plate 156 and the reticle plate 157, refer to, for example, JP2004-229769A.

The half mirror 154 is arranged in the lateral direction (X direction) of the subject eye E. The half mirror 154 transmits or reflects the illumination light beam emitted to the subject eye E and reflected by the subject eye E. The imaging element 155 is arranged in the transmission direction of the half mirror 154.

The imaging element 155 captures the cornea of the subject eye E from the side direction via the half mirror 154 and the first observation window 152. For example, a captured image (second image) captured by the imaging element 155 may be transmitted to the controller 200 via the shared server 300 and may be displayed on the monitor 220 included in the controller 200. For example, the examiner D can understand the positional relationship between the subject eye E and the measurement unit 120 by observing the positions of the subject eye E and the scale lines that is not shown of the targeting scale plate 156, using the second image. Imaging by the imaging element 155 is controlled by the control unit 170.

For example, the assistant A (assistant eye AE) can understand the positional relationship between the subject eye E and the measurement unit 120 by looking into the second observation window 153 and observing the subject eye E through the half mirror 154 and the first observation window 152.

### <Examiner controller>

For example, the controller 200 includes a switch unit 210, the monitor 220, the control unit 280 (refer to FIG. 9), and the like.

The switch unit 210 is an operation unit for operating the optometry device 100. For example, the switch unit 210 can change the display of the visual target on the display 161. Further, for example, the switch unit 210 can change at least one of the position of the forehead pad 121, the movement of the lens unit 130, the rotation angle of the lens disk 131, the rotation angle of the optical element 133, and the like in the measurement unit 120. A signal corresponding to the operation instruction from the switch unit 210 is transmitted to the optometry device 100 via the shared server 300.

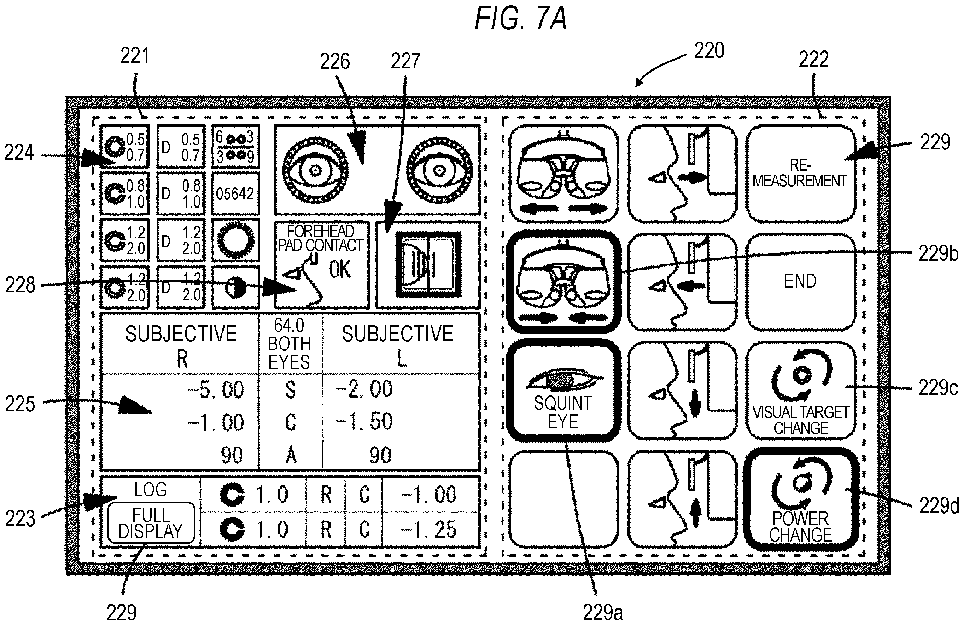

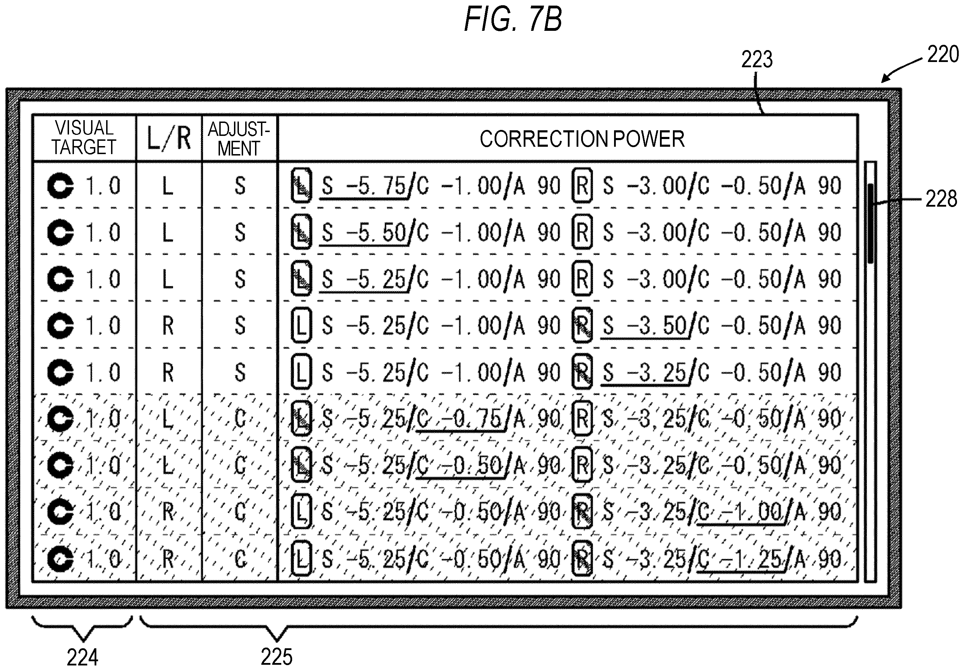

The monitor 220 displays various types of information. The monitor 220 may be a touch panel, and the monitor 220 may also function as an operation unit. The monitor 220 can display at least one of the first operation screen, the first instruction screen, the data log screen, and the like (refer to FIG. 7). For example, the first operation screen is used for inputting an operation signal for controlling display of visual target on the display 161, switching between the lens disk 131 or the optical element 133 in the measurement unit 120, and the like. Further, for example, the first operation screen is used to confirm the measurement result of the subject eye E. For example, the first instruction screen is used to input a trigger signal that serves as a trigger for transmitting suggestions or instructions to the assistant A. For example, the data log screen is used to confirm a data log (hereinafter referred to as a log) indicating the state of the optometry system 1 in a case where the display 161 or the measurement unit 120 is controlled based on the operation signal described above.

The monitor 220 may display all screens such as the first operation screen, the first instruction screen, the data log screen, and the like in the display area thereof. Further, the monitor 220 may display the first operation screen, the first instruction screen, the data log screen, and the like one by one at a time in the display area thereof. That is, these screens may be displayed in a switchable manner. It is needless to say that the monitor 220 may display a combination of any of the screens such as the first operation screen, the first instruction screen, the data log screen, and the like in the display area thereof.

FIGS. 7A and 7B are examples of the monitor 220. FIG. 7Ais an example of a first operation screen 221, the first instruction screen 222, and a data log screen 223. FIG. 7B is an example of the data log screen 223. For example, in the present example, the data log screen 223 shown in FIG. 7A displays some logs, and the data log screen 223 shown in FIG. 7B displays all of the logs. For example, by switching the display form of the data log screen 223, it is possible to change partial display and full display of the logs to each other (details will be described later).

A visual target switching switch 224, a result image 225, a first image 226, a second image 227, a third image 228, and the like may be displayed on the first operation screen 221 shown in FIG. 7A.

The visual target switching switch 231 is a switch for changing the visual target to be displayed on the display 161. An operation signal input from the visual target switching switch 231 is transmitted to the optometry device 100 by the control unit 280. The result image 225 is an image showing the examination result of the subjective examination for the subject eye E. The first image 226 is an image of the subject eye E captured from the front by the imaging element 165 of the light projecting optical system 160. The second image 227 is an image of the subject eye E captured from the side direction by the imaging element 155 of the targeting unit 150. The third image 228 is an image showing the detection result obtained by detecting the contact between the head portion of the examinee and the forehead pad 121 by the detector 123 provided in the forehead pad 121.

An instruction input switch 229 may be displayed on the first instruction screen 222 shown in FIG. 7A. The instruction input switch 229 is a switch for inputting an instruction to the assistant A. A trigger signal input from an instruction switch 291 is transmitted to the controller 400 by the control unit 280.

The instruction input switch 291 may include a switch for instructing an action related to the measurement posture in a case where the examinee S observes the presented visual target. Using such a switch, instructions for improving the deviation between the subject eye E and the measurement unit 120 (for example, the position of the forehead pad 121 and the distance between the lens units 130), the eyelid-open state of the subject eye E (for example, squint state), and the like may be input. Further, the instruction switch 291 may include a switch for the assistant A to instruct an operation related to a procedure for changing the display of the visual target, a procedure for switching the lens disk 131 or the optical element 133, and the like. Using such a switch, instructions for prompting the change of the visual target, the change in correction power, the change in adjustment item (which will be described later), repeating of the measurement, skipping of the measurement, end of the measurement, and the like, may be input.

For example, each instruction input switch may be represented by an icon that symbolizes the contents of instructions to the assistant A with simple pictures or characters. Further, for example, the trigger signal input from each instruction switch may be associated with instruction information 492 that will be described later.

The data log screen 223 shown in FIG. 7B may include a log indicating at least one of the log indicating the visual target information 230 and the log indicating the correction information 231.

The visual target information 224 may be information related to the visual target presented to the subject eye E. For example, the visual target information 224 may be information indicating the type of the visual target displayed on the display 161. Further, for example, the visual target information 224 may be information indicating the visual acuity value of the visual target displayed on the display 161. It is needless to say that the visual target information 224 may be information indicating both the type of the visual target and the visual acuity value of the visual target.

The correction information 225 may be any information related to the correction of the subject eye E. For example, the correction information 231 may be information indicating the target eye (either the left eye or the right eye) of which the optical element 133 corrects the subject eye E. Further, for example, the correction information 231 may be information indicating the adjustment item of which the correction power is adjusted with respect to the subject eye E. Further, for example, the correction information 231 may be information indicating a correction power by which the subject eye E is corrected. It is needless to say that the correction information 225 may be information indicating at least one of the target eye, the adjustment item, and the correction power.

The instruction information 224 or the correction information 225 may be highlighted or color-coded. For example, the correction power corresponding to the target eye and the adjustment item may be highlighted. As an example, in a case where the spherical power of the left eye is adjusted, the value of the corresponding spherical power may be emphasized and displayed. Further, for example, the instruction information 224 or the correction information 225 may be color-coded according to the type of the visual target, the adjustment item, and the like. As an example, the instruction information 224 or the correction information 225 may be color-coded according to each adjustment item for the spherical power, the cylindrical power, and the astigmatism axis angle.

A scroll bar 228 may be displayed on the data log screen 223. A scroll bar 228 is displayed in a case where each log does not fit in the display area. For example, by sliding the scroll bar 228, a log hidden outside the display area can be displayed.

### <Assistant controlled

For example, the controller 400 includes a switch unit 410, the monitor 420, a control unit 450 (refer to FIG. 9), and the like.

The switch unit 410 is an operation unit for operating the optometry device 100. For example, the switch unit 410 can change the display of the visual target on the display 161. Further, for example, the switch unit 410 can change at least one of the position of the forehead pad 121, the movement of the lens unit 130, the rotation angle of the lens disk 131, the rotation angle of the optical element 133, and the like in the measurement unit 120. A signal corresponding to the operation instruction from the switch unit 410 is transmitted to the optometry device 100 via the shared server 300.

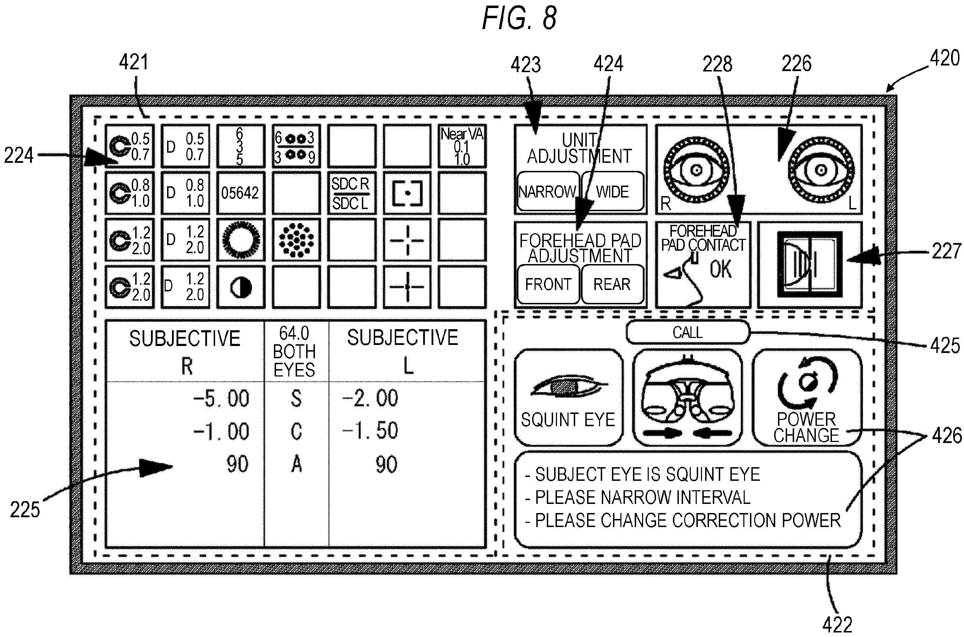

The monitor 420 displays various types of information. The monitor 420 may be a touch panel, and the monitor 420 may also function as the switch unit 410. The monitor 420 can display at least one of the second operation screen, the second instruction screen, and the like (refer to FIG. 8).

For example, the second operation screen is used for inputting operation signals for controlling display of visual target on the display 161, switching between the lens disk 131 or the optical element 133 in the measurement unit 120, and the like. Further, for example, the second operation screen is used for inputting operation signals for controlling the movement of the measurement unit 120 with respect to the subject eye E, and the like. Further, the second operation screen is used to confirm the measurement result of the subject eye E. For example, the second instruction screen is used to confirm suggestions or instructions from the examiner D. Further, for example, the second instruction screen is used by the assistant A to make a request such as an inquiry to the examiner D.

The monitor 420 may display both the second operation screen and the second instruction screen on the display area thereof. Further, the monitor 420 may display a combination of the second operation screen and the second instruction screen on the display area thereof. That is, these screens may be displayed in a switchable manner.

FIG. 8 is an example of a monitor 440. For example, in the present example, both the second operation screen 421 and the second instruction screen 422 are displayed in the display area of the monitor 420.

The visual target switching switch 224, the result image 225, the first image 226, the second image 227, the third image 228, and the like, which are described above, may be displayed on the second operation screen 421. A unit adjustment switch 423 and a forehead pad adjustment switch 424 may also be displayed on the second operation screen 421. The unit adjustment switch 423 is a switch for changing the interval between the lens units 130. The forehead pad adjustment switch 424 is a switch for changing the position of the forehead pad 121. The operation signals input from these switches are transmitted to the optometry device 100 by the control unit 450.

The instruction information screen 422 may display a request switch 425, instruction information 426, and the like. The request switch 425 is a switch for requesting assistance from the examiner D. In other words, the request switch 425 is a switch for causing the examiner controller 200 to output instruction information corresponding to the trigger signal input from the assistant controller 400. A trigger signal input from the request switch 425 is transmitted to the controller 200 by the control unit 450.

The instruction information 426 is information indicating instruction contents with respect to an action related to at least one of the measurement posture of the examinee S and the measurement procedure of the assistant A, which are associated with the trigger signal input from the instruction input switch 291. For example, the instruction information 426 may be represented by an icon that symbolizes the instruction contents from the examiner D with simple pictures or characters. Further, for example, the instruction information 426 may be represented by a message indicating the instruction contents from the examiner D. It is needless to say that the instruction information 426 may be a combination of these.

### <Control system>

FIG. 9 is a view showing a control system of the optometry system 1. For example, the shared server 300 includes a control unit 301, a memory 302, and the like.

The control unit 301 is configured by a general CPU, RAM, ROM, and the like. For example, the CPU may control communication from the controller 200 and the controller 400 with respect to the optometry device 100, communication from the optometry device 100 and the controller 400 with respect to the controller 200, communication from the optometry device 100 and the controller 200 with respect to the controller 400, and the like. For example, the RAM temporarily stores various pieces of information. For example, the ROM may store a program for controlling the operation of the optometry device 100, a program for controlling the operation of the controller 200, and the like.

The control unit 170 in the optometry device 100, the control unit 280 in the controller 200, the control unit 450 in the controller 400, and the like are electrically connected to the control unit 301. The display 161, the imaging element 165, and the like in the light projecting optical system 160 are electrically connected to the control unit 170. In addition, each driving unit (the driving unit 122, the driving unit 134, the driving unit 135, the driving unit 141, and the driving unit 142), the detector 123, the imaging element 155, and the like in the measurement unit 120 are electrically connected to the control unit 170. The switch unit 210, the monitor 220, and the like are electrically connected to the control unit 280. The switch unit 410, the monitor 420, and the like are electrically connected to the control unit 450. It is needless to say that each member of the controller 400, each member of the photographing camera 500, each member of the monitor 600, and the like may be electrically connected to the control unit 301.

The memory 302 is a non-transitory storage medium that can hold stored contents even in a case where power supply is interrupted. For example, as the memory 302, a hard disk drive, a flash ROM, a USB memory, or the like can be used. For example, the memory 302 may store visual target data to be displayed on the display 161, a first image 260 captured by the imaging element 165, a second image 270 captured by the imaging element 155, and the like.

### <Action>

An action in which the assistant A performs a subjective examination on the examinee S (subject eye E) using the optometry system 1 will be described.

### <Alignment of subject eye and measurement unit>

When starting the subjective examination of the subject eye E, alignment of the subject eye E and the measurement unit 120 is performed. The assistant A instructs the examinee S to bring the face into contact with the forehead pad 121 and observe the display 161 through the optometry window 132 and the presentation window 111.

At this time, the control unit 170 of the optometry device 100 transmits the first image 226 obtained by capturing the subject eye E from the front, the second image 227 obtained by capturing the subject eye E from the side, and the third image 228 indicating the detection result of the detector 123, to the control unit 280 of controller 200 and the control unit 450 of controller 400. The control unit 280 receives each image and displays each image on the monitor 220. Further, the control unit 450 receives each image and displays each image on the monitor 420 (second operation screen 421). As a result, the examiner D and the assistant A can confirm the first image 226, the second image 227, and the third image 228.

The assistant A measures the pupillary distance of the examinee S in advance and inputs the value using the controller 400. The control unit 170 of the optometry device 100 drives the driving unit 141 to move the lens unit 130 in the left-right direction (X direction). Here, the assistant A observes the first image 226 or looks into the observation window 113 to confirm the position of the center of the pupil of the subject eye E and the position of the optometry window 132. Further, the assistant A operates the unit adjustment switch 423 as necessary to adjust the interval between the lens units 130 with respect to the center position of the pupil of the subject eye E. Accordingly, the optometry window 132 is matched with the pupillary distance of the subject eye E.

Furthermore, the assistant A observes the second image 227 or looks into the second observation window 153 to confirm the corneal apex position of the subject eye E and the position of the optometry window 132. Further, the assistant A operates the forehead pad adjustment switch 424 as necessary to adjust the position of the forehead pad 121. The control unit 170 of the optometry device 100 drives the driving unit 122 to move the forehead pad 121 in the front-rear direction (Z direction), and changes the position of the forehead pad 121 with respect to the optometry window 132. As a result, the corneal apex position of the subject eye E is matched with the reference position when the lens is worn.

### <Subjective examination for subject eye>

In a case where the alignment of the subject eye E and the measurement unit 120 is completed, the subj ective examination of the subject eye E is started. The assistant A operates at least one of the switch unit 410 and the monitor 420 (second operation screen 421) to set a predetermined visual target and a desired correction power. For example, the assistant A may operate the visual target switching switch 231 to set the type of the visual target and the visual acuity value of the visual target. Further, for example, the assistant A may operate the switch unit 410 to set a desired correction power (for example, 0D). The control unit 450 of the controller 400 transmits, to the optometry device 100, an operation signal input from at least one of the switch unit 410 and the monitor 420 (second operation screen 421). The control unit 170 of the optometry device 100 controls the measurement unit 120 based on the operation signal.

For example, the control unit 170 controls the driving unit 134 to change the rotation angle of the lens disk 131, and arranges the optical element 133 having a predetermined spherical power in the optometry window 132. Further, for example, the control unit 170 controls the driving unit 134 to change the rotation angle of the lens disk 131, and arranges the optical element 133 having a predetermined cylindrical power in the optometry window 132. Further, for example, the control unit 170 controls the driving unit 135 to arrange the optical element 133 having a predetermined cylindrical power in the optometry window 132 at a predetermined rotation angle. As a result, the subject eye E is corrected such that the target light flux from the display 161 is focused on the retina via the optical element 133 (that is, the eye refractive power of the subject eye E becomes 0D).

The assistant A confirms whether or not the correction power for correcting the subject eye E is appropriate while operating the visual target switching switch 231 to switch the visual target presented to the subject eye E. In a case where the correction power to correct the subject eye E is inappropriate, the eye refractive power of the subject eye E may be corrected with a value different from 0D, and it may be confirmed again whether or not the correction power is appropriate. At this time, the logs indicating the type and the visual acuity value of the visual target presented to the subject eye E by the assistant A, the correction power added to the subject eye E, the adjustment item for the correction power, the target eye of the measurement, and the like, are sequentially stored (accumulated) in the memory 302.

### <Communication between examiner and assistant>

For example, in a case where the assistant A is unfamiliar with the subjective examination, the assistant A may request assistance from the examiner D, and the examiner D may ask the assistant A to confirm the measurement situation. However, since the assistant A and the examiner D are in separate rooms, it is difficult for them to communicate with each other.

Therefore, in the present example, the optometry system is constructed such that the instruction information 426 can be transmitted from the controller 200 operated by the examiner D to the controller 400 operated by the assistant A. As a result, even in a case where the assistant A and the examiner D are apart from each other, they can easily request assistance, understand the measurement situation, share information, and instruct the next operation.

The assistant A operates the controller 400 and selects the request switch 425 in a case where the examiner D needs assistance. The control unit 450 of the controller 400 transmits the trigger signal input from request switch 425 to the control unit 280 of the controller 200. For example, based on the trigger signal, the control unit 280 generates instruction information indicating that the assistant A is requesting assistance, from a speaker that is not shown, as a voice guide.

In response to the request from the assistant A, the examiner D confirms the first operation screen 221 and the data log screen 223 in order to understand the measurement situation of the subject eye E. For example, the examiner D confirms at least one of the first image 226, the second image 227, and the third image 228 on the first operation screen 221 in order to understand the measurement posture in a case where the examinee S observes the presented visual target. Further, for example, the examiner D confirms at least one of the visual target information 224 and the correction information 225 on the data log screen 223 in order to understand the procedure in which the assistant A switched the visual target or the optical element 133. Further, the examiner D determines the next operation to be performed by the assistant A according to the measurement situation of the subject eye E, and appropriately operates the first instruction screen 222 to instruct the assistant A.

First, understanding of the measurement posture of the examinee S and instruction of the next operation will be described.

As an example, the examiner D uses the first image 226 to confirm the eyelid-open state of the subject eye E, and selects the corresponding icon from the instruction input switch 229 in a case where the subject eye E is a squint eye. For example, the user selects an icon 229a (refer to FIG. 8) representing the content notifying that the subject eye is a squint eye. Further, as an example, the examiner D uses the first image 226 to confirm whether or not the center position of the pupil of the subject eye E and the center position of the optometry window 132 substantially match each other. In a case where the center position of the pupil of the subject eye E and the center position of the optometry window 132 does not match each other, a corresponding icon is selected from the instruction switch 291. For example, an icon 229b (refer to FIG. 8) representing the content of narrowing the interval between the lens units 130 is selected.

The control unit 280 of the controller 200 transmits the trigger signal input from the icon 229a and the icon 229b to the control unit 450 of the controller 400. The control unit 450 causes the instruction information screen 422 to display the instruction information 426 indicating the instruction content from the examiner D, which is associated with these trigger signals. For example, the same illustration as the icon 229a and a message indicating that the subject eye is a squint eye may be displayed. Further, for example, the same illustration as the icon 229b and a message indicating that the interval between the lens units 130 should be narrowed may be displayed.

The assistant A can recognize that the measurement posture of the examinee S is inappropriate by confirming the instruction information 426. In addition, the assistant A can issue appropriate instructions to the examinee S to improve the squint eye, and arrange the measurement unit 120 at an appropriate position with respect to the examinee S (subject eye E).

In the above description, the case where the examiner D uses the first image 226 to determine the deviation between the subject eye E and the optometry window 132 in the left-right direction (X direction) is taken as an example, but the present invention is not limited thereto. The examiner D may use the second image 227 to determine the deviation between the subject eye E and the optometry window 132 in the front-rear direction (Z direction). Further, the examiner D may use the third image 228 to determine whether or not the head portion of the examinee is in contact with the forehead pad 121. For example, even in these cases, the examiner D can select a predetermined icon to notify the assistant A of this fact and instruct the assistant A to perform the next operation.

Next, understanding about the procedure for switching the visual target or the optical element by the assistant A and instructions for the next operation will be described.

As an example, the examiner D uses the visual target information 224 to confirm the transition of the type and the visual acuity value of the visual target presented to the subject eye E, and in a case where the transition is inappropriate, a corresponding icon is selected from the instruction input switch 229. For example, an icon 229c representing the content prompting to change the visual target is selected. Further, as an example, the examiner D uses the correction information 225 to confirm the transition of the correction power by which the subject eye E is corrected, and in a case where the correction power is inappropriate, a corresponding icon is selected from the instruction input switch 229. For example, an icon 229d representing the content prompting to change the correction power is selected.

The control unit 280 of the controller 200 transmits the trigger signal input from the icon 229c and the icon 229d to the control unit 450 of the controller 400. The control unit 450 causes the instruction information screen 422 to display the instruction information 426 indicating the instruction content from the examiner D, which is associated with these trigger signals. For example, the same illustration as the icon 229c and a message indicating that the visual target should be changed may be displayed. Further, for example, the same illustration as the icon 229d and a message indicating that the correction power should be changed may be displayed.

By confirming the instruction information 426, the assistant A can recognize that the setting of the visual target or the correction power by the assistant A is inappropriate. In addition, the assistant A can appropriately change the visual target or the correction power, and perform measurement again.

### <Confirmation of data log by examiner>

The examiner D may confirm with the assistant A how the assistant A proceeded with the measurement depending on the measurement situation of the subject eye E. For example, in a case where the change in the subjective value of the subject eye E does not gradually decrease, or the like, the examiner D may confirm the flow of a series of measurements by the assistant A. However, the examiner D and the assistant A are in separate rooms, and it is difficult for the examiner D to understand the measurement action of the assistant A.

For this reason, in the present example, the optometry system is constructed such that a log indicating the state of the optometry system can be displayed on the controller 200 operated by the examiner D. For example, the log indicating the state of the optometry system may be a log indicating the state of the system in a case where the display of the display 161 is controlled by the control unit 170 of the optometry device 100. Further, the log indicating the state of the optometry system may be a log indicating the state in a case where at least one of the rotation angle of the lens disk 131, the rotation angle of the optical element 133, and the like is controlled by the control unit 170. These logs may be analyzed such that each state can be easily recognized.

For example, in the present example, on the data log screen 223 shown in FIG. 7A, a log showing the state of the optometry system before the control unit 170 performs a predetermined control (that is, a state one operation before), and a log showing the state of the optometry system after the control unit 170 performs a predetermined control, are displayed. These logs may be some logs in the information included in the visual target information 224 and the information included in the correction information 225. For example, in the present example, both the type and the visual acuity value of the visual target are displayed as the visual target information 224, but only the correction power value (that is, one of the spherical power value, the cylindrical power value, and the astigmatism axis angle) corresponding to the target eye and the adjustment item may be displayed as the correction information 225.

The examiner D uses the data log screen 223 to confirm the log indicating the visual target information 224 and the correction information 225 and understands the measurement situation of the subject eye E from the information such as the type and the visual acuity value of the visual target presented to the subject eye E by the assistant A, the correction power value added to the subject eye E, the adjustment item of which the correction power value is adjusted, and the target eye of the measurement. For example, by confirming the data log screen 223 together with the first operation screen 221, the examiner D can understand the suitability of the visual target presented by the assistant A, the suitability of the set correction power, and the like.

For example, the examiner D may operate either the switch unit 210 or the monitor 220 to select the screen switching switch 229. The control unit 280 of the controller 200 switches the display mode of the data log screen 223 on the monitor 220, based on the switching signal input from the screen switching switch 229. For example, a folding display mode, as shown in FIG. 7A, for displaying a part of the information included in the visual target information 224 and the correction information 225, and a full display mode, as shown in FIG. 7B, for displaying all of the information included in the visual target information 224 and the correction information 225, are switched to each other. As an example, in the full display mode, the spherical power values, the cylindrical power values, and the astigmatism axis angles with respect to each of the left eye and the right eye are all displayed.

The examiner D may operate the scroll bar 228 to confirm the log indicating the visual target information 224 and the correction information 225 as a whole, and understand the measurement situation of the subject eye E. For example, in addition to the suitability of the visual target presented by the assistant A and the suitability of the set correction power, the examiner D can understand the suitability of the procedure for switching the visual target or the correction power, the timing or frequency of switching the visual target or the correction power, and the like. The examiner D may select the corresponding icon from the instruction input switch 229 described above and instruct the assistant Ato change or re-measure the visual target or the correction power.

As described above, for example, in the optometry system according to the present example, the first trigger signal is transmitted from the first operation means to the second operation means in response to an operation with respect to the first instruction screen in the first operation means, and the second operation means outputs the first instruction information for assisting the action of the optometry system based on the first trigger signal transmitted from the first operation means. As a result, even when the examiner is apart from the operator (assistant), the examiner can easily convey the contents of the suggestions or instructions, and the subjective examination can be accurately performed.

Further, for example, in the optometry system according to the present example, the second operation means outputs, as the first instruction information, at least the instruction information for assisting the measurement posture in a case where the examinee observes the visual target presented by the visual target presenting means. By confirming the first instruction information, the examiner can determine the quality of the measurement posture of the examinee, and to convey appropriate instructions to the operator.

Further, for example, in the optometry system according to the present example, the second operation means outputs, as the first instruction information, at least the instruction information for assisting the procedure of operating at least one of the visual target presenting means and the correction means. By confirming the first instruction information, the examiner can determine the quality of the operation procedure by the assistant, and can convey appropriate instructions to the operator.

Further, for example, in the optometry system according to the present example, at least one sign associated with the first trigger signal is displayed on the first instruction screen, and the first trigger signal corresponding to the sign is transmitted from the first operation means to the second operation means, based on an operation with respect to the at least one sign. For example, the examiner can easily transmit an appropriate trigger signal simply by selecting a sign since the sign is associated with the first trigger signal.

Further, for example, in the optometry system according to the present example, the second instruction screen is displayed on the second operation means, the second trigger signal is transmitted from the second operation means to the first operation means in response to an operation with respect to the second instruction screen in the second operation means, and the first operation means and the second operation means communicate with each other. As a result, the examiner and the operator (assistant) can send instruction information to each other and easily convey the contents of the suggestions or instructions to each other.

Further, for example, the optometry system according to the present example acquires a data log indicating the state of the optometry system in a case where at least one of the visual target presenting means and the correction means is controlled, and outputs this. The examiner can easily confirm the suitability of the subjective examination by the operator even when the examiner is apart from the operator (assistant) who is unfamiliar with the examination. Further, the examiner can easily determine whether to re-execute the examination or the like.

Further, for example, the optometry system according to the present example acquires a data log of at least one of the visual target information related to the visual target presented to the subject eye and the correction information related to correction of the subject eye. As a result, the examiner can easily determine whether or not the visual target presented to the subject eye is appropriate, or whether or not the correction state of the subject eye is appropriate.

Further, for example, the optometry system according to the present example acquires a data log of at least one of the type of the visual target and the visual acuity value of the visual target, as visual target information related to the visual target presented to the subject eye. Accordingly, the examiner can easily determine whether or not the type and the visual acuity value of the visual target presented to the subject eye are appropriate.

Further, for example, the optometry system according to the present example acquires, as correction information related to the correction of the subject eye, a data log of at least one of the target eye of the subjective examination, the adjustment item of which the correction power is adjusted with respect to the subject eye, and the correction power value by which the correction means corrects the subject eye. Accordingly, the examiner can easily determine which correction power value is set for each adjustment item for the left eye and the right eye.

Further, for example, the optometry system according to the present example switches from the output of the first data log that is a part of the data log to the output including at least the second data log different from the first data log, according to the switching signal for switching the output form by the output means. As a result, for example, the examiner can easily confirm the data log hidden outside the display screen, the data log displayed in a folded state, and the like.

### <Modification example>

In the present example, although a configuration is adopted in which the instruction information 426 can be transmitted from the controller 200 operated by the examiner D to the controller 400 operated by the assistant A, and the instruction information (here, a voice guide indicating that the assistant A is requesting assistance) can be transmitted from the controller 400 to the controller 200, the present invention is not limited thereto. For example, in the present example, at least the configuration may be adopted in which the instruction information can be transmitted from the controller 200 to the controller 400.

Further, in the present example, a configuration in which the controller 200 operated by the examiner D displays the first instruction screen 222 used for inputting instructions from the examiner D to the assistant A, and the controller 400 operated by the assistant A displays the second instruction screen 422 used for outputting the instruction information 426 from the examiner D has been described as an example, but the present invention is not limited thereto. For example, the controller 200 may display an instruction screen used for outputting instruction information from the assistant A together with the first instruction screen 222. Further, for example, the controller 400 may display an instruction screen used for inputting instructions from the assistant A to the examiner D together with the second instruction screen 422. That is, the controller 200 and the controller 400 may each be provided with an instruction screen used for inputting an instruction to the other party and an instruction screen used for outputting an instruction from the other party.

For example, in a case of the above configuration, the examiner D and the assistant A select the icon indicating the instruction content to the other party displayed on the screen of each controller, and accordingly, the predetermined instruction information can be displayed on the screen of the controller of the other party. As an example, the examiner D can display instruction information or the like for instructing the assistant A to perform the next action. Further, as an example, the assistant A can display instruction information or the like for asking the examiner whether or not the action of the assistant A is appropriate.

In the present example, a configuration in which the trigger signal is transmitted to the controller 400 by the examiner D directly operating the first instruction screen 222 (more specifically, selecting the instruction input switch 229 displayed on the first instruction screen 222) has been described as an example, but the present invention is not limited thereto. In the present example, the configuration may be adopted in which the trigger signal is transmitted to the controller 400 by the examiner D indirectly operating the first instruction screen 222.

In this case, the first instruction screen 222 may be provided with a writing area in which any character can be written with a finger or a touch pen. Further, in this case, the first instruction screen 222 may be provided with a typing area in which any character can be typed using a keyboard or the like. Further, in this case, the first instruction screen 222 may be provided with a voice conversion area that converts voice from a microphone or the like into characters and displays the characters. For example, the control unit 280 may identify characters input in the writing area, the typing area, and the voice conversion area, and transmit the characters to the controller 400 as operation signals.

Note that, in the present example, a configuration in which an icon prepared in advance as the instruction input switch 229 is displayed on the first instruction screen 222 has been described as an example, but the present invention is not limited thereto. In the present example, the first instruction screen 222 may be configured to display only predetermined icons among the icons prepared in advance as the instruction input switch 229. In this case, only any icon designated by the examiner D may be displayed. Further, in this case, an icon may be displayed according to each measurement content with respect to the subject eye E. For example, an icon preset for each examination mode may be displayed in response to switching of the examination mode by the examiner D or the assistant A, switching of the examination mode by programmed optometry, or the like.

Further, in the present example, it may be possible to set the alignment order of the icons prepared as the instruction input switch 229. For example, the alignment of icons may be set manually. In this case, the examiner D associates the icons representing the contents of each instruction with the display positions of the icons on the first instruction screen 222 in advance and stores them in the memory 302, and accordingly, the alignment of the icons may be set. Further, for example, the alignment of icons may be set automatically. In this case, the alignment of icons may be set based on the frequency of use of the icons.

For example, in the present example, it is possible to set the display contents and the alignment order of display of the signs on the first instruction screen 222 in this manner. Accordingly, the examiner easily selects the necessary signs according to the examination items, frequency of use for the subject eye, and the operability is improved.

In the present example, a configuration in which the visual target information 224 and the correction information 225 are displayed on the data log screen 223 has been described as an example, but the present invention is not limited thereto. In the present example, a log showing names of examination items that the assistant A performed with respect to the subject eye E may be displayed together with these pieces of information. For example, in a case where the assistant A performs the programmed optometry on the subject eye E, the preset examination items sequentially proceed. At this time, the control unit 170 of the optometry device 100 associates each examination item name with the visual target and the correction power changed in each examination item, and stores them in the memory 302. For example, the control unit 280 of the controller 200 may call the log indicating the examination item name and the log indicating the visual target information 224 and the correction information 225 from the memory 302, and display them on the data log screen 223. In addition, in the optometry program, in a case where the examination item name is associated with the order in which each examination item proceeds, a log indicating the examination number may be displayed.

In the present example, a configuration in which at least one of the spherical power, the cylindrical power, and the astigmatism axis angle is adjusted with respect to the subject eye E, and symbols corresponding to these are displayed as adjustment item information in the correction information 225 has been described as an example, but the present example is not limited thereto. It is needless to say that items other than these may be adjusted with respect to the subject eye E, and corresponding symbols may be displayed as the adjustment item information. As an example, a prism value may be adjusted with respect to the subject eye E, and a symbol such as "P" corresponding to the prism value may be displayed as the adjustment item information. At this time, as the correction power information in the correction information 225, the prism value may be displayed together with the spherical power or the like, or only the prism value may be displayed.

In the present example, a configuration in which all of the logs indicating the state in a case where at least one of the display of the display 161, the rotation angle of the lens disk 131, the rotation angle of the optical element 133, and the like is controlled by the operation of the assistant A are displayed on the data log screen 223, has been described as an example, but the present invention is not limited thereto. For example, a configuration may be adopted in which a specific log is extracted from such logs and only the specific log is displayed on the data log screen 223.

The specific log may be a log indicated in units of one operation by the assistant A. For example, the log may be a log showing a unit of one operation in which the assistant A switches one of the type of the visual target, the visual acuity value of the visual target, the correction power (the spherical power, the cylindrical power, and the astigmatism axis angle), and the like. That is, the log may be obtained each time the control unit 170 controls any one of the display of the display 161, the rotation angle of the lens disk 131, the rotation angle of the optical element 133, or the like.

Further, the specific log may be a log indicated in units of one examination by the assistant A. For example, the log may be a log indicated in units of one examination including operations performed by the assistant A from the start to the end of a predetermined examination item (for example, the spherical test, the astigmatism test, and the like). That is, the log may be a set of logs obtained each time any one of the display of the display 161, the rotation angle of the lens disk 131, the rotation angle of the optical element 133, and the like is controlled from the start to the end of a predetermined examination item.

The specific log may include a log at least one operation before (or one examination item before), after the operation (or examination item) at the timing at which the assistant A requested assistance from the examiner D. In other words, the specific log may be only the log one operation before (or one examination item before), or may be a range from the log one operation before (or one examination item before) to the log before any operation (or before the examination item). In this case, the specific log range may be designated by the examiner D in any manner, or may be set in advance to a predetermined number of operations (or the number of examination items).

For example, by extracting a specific log and displaying the specific log on the monitor 220 in this manner, the examiner can confirm the suitability of the spherical surface information (for example, the spherical power and the like) or the astigmatism information (for example, the cylindrical power, the astigmatism axis angle, and the like) of the subject eye, the suitability of the spherical test for obtaining the spherical information or the astigmatism test for obtaining the astigmatism information, or the like.

### REFERENCE SIGNS LIST

1 Optometry system
100 Subjective optometry device
120 Eye refractive power measurement unit
160 Display
200 Optometry controller
220 Monitor
223 Log image
230 Operation image
300 Shared server
400 Assistant controller

## Claims

1. An optometry system for subjectively measuring an optical characteristic of a subject eye, the optometry system comprising:
a visual target presenting means that emits a target light flux toward the subject eye;
a correction means that changes an optical characteristic of the target light flux;
an operation means for operating at least one of the visual target presenting means and the correction means;
a control means that controls at least one of the visual target presenting means and the correction means in response to an operation with respect to the operation means;
an acquisition means that acquires a data log indicating a state of the optometry system in a case where the control means controls at least one of the visual target presenting means and the correction means; and
an output means that outputs the data log.

2. The optometry system according to claim 1,
wherein the data log is a data log of at least one of visual target information related to a visual target presented to the subject eye and correction information related to correction of the subject eye.

3. The optometry system according to claim 2,
wherein the data log of the visual target information is a data log of at least one of a type of the visual target and a visual acuity value of the visual target.

4. The optometry system according to claim 2,
wherein the data log of the correction information is a data log of at least one of a target eye of the subjective examination on the subject eye, an adjustment item of which a correction power is adjusted with respect to the subject eye, and a correction power value by which the correction means corrects the subject eye.

5. The optometry system according to any one of claims 1 to 4, further comprising:
an extraction means that extracts a specific data log from the data log acquired by the acquisition means,
wherein the output means outputs the specific data log extracted by the extraction means.

6. The optometry system according to claim 5,
wherein the output means switches from an output of a first data log indicating a part of the data log to an output including at least a second data log different from the first data log indicating a part of the data log, according to a switching signal that switches an output form by the output means.

7. An optometry system for subjectively measuring an optical characteristic of a subject eye, the optometry system comprising:
a visual target presenting means that emits a target light flux toward the subject eye;
a correction means that changes an optical characteristic of the target light flux;
a first operation means for operating at least one of the visual target presenting means and the correction means;
a second operation means different from the first operation means; and
a display control means that causes a display means of the first operation means to display a first instruction screen for transmitting, to the second operation means, a first trigger signal for controlling an action of the second operation means,
wherein the first trigger signal is transmitted from the first operation means to the second operation means in response to an operation with respect to the first instruction screen in the first operation means, and
the second operation means outputs first instruction information for assisting an action of the optometry system, based on the first trigger signal transmitted from the first operation means.

8. An optometry system for subjectively measuring an optical characteristic of a subject eye, the optometry system comprising:
a visual target presenting means that emits a target light flux toward the subject eye;
a correction means that changes an optical characteristic of the target light flux;
a first operation means;
a second operation means for operating at least one of the visual target presenting means and the correction means, the second operation means being different from the first operation means; and
a display control means that causes a display means of the first operation means to display a first instruction screen for transmitting, to the second operation means, a first trigger signal for controlling an action of the second operation means,
wherein the first trigger signal is transmitted from the first operation means to the second operation means in response to an operation with respect to the first instruction screen in the first operation means, and
the second operation means outputs first instruction information for assisting an action of the optometry system, based on the first trigger signal transmitted from the first operation means.

9. The optometry system according to claim 7 or 8,
wherein the first instruction information includes at least instruction information for assisting a measurement posture in a case where the examinee observes a visual target presented by the visual target presenting means.

10. The optometry system according to any one of claims 7 to 9,
wherein the first instruction information includes at least instruction information for assisting a procedure for operating at least one of the visual target presenting means and the correction means.

11. The optometry system according to any one of claims 7 to 10,
wherein the display control means causes the first instruction screen to display at least a sign associated with the first trigger signal, and
the first operation means transmits, to the second operation means, the first trigger signal according to the sign from the first operation means, based on an operation with respect to the at least one sign.

12. The optometry system according to claim 11, further comprising:
a setting means that sets an arrangement of the sign,
wherein the display control means rearranges the sign on the first instruction screen, based on the arrangement set by the setting means.

13. The optometry system according to claims 7 to 12,
wherein the display control means causes a display means of the second operation means to display a second instruction screen for transmitting, to the first operation means, a second trigger signal for controlling an action of the first operation means,
the second trigger signal is transmitted from the second operation means to the first operation means in response to an operation with respect to the second instruction screen in the second operation means,
the first operation means outputs second instruction information for assisting an action of the optometry system, based on the second trigger signal transmitted from the second operation means, and
the first operation means and the second operation means are capable of communicating with each other.

14. The optometry system according to any one of claims 7 to 13,
wherein the display control means causes the display means of at least one of the first operation means and the second operation means to display an operation screen for transmitting an operation signal for controlling at least one of the visual target presenting means and the correction means.

15. An optometry controller that operates an optometry system for subjectively measuring an optical characteristic of a subject eye, the optometry system including a visual target presenting means that emits a target light flux toward the subject eye, and a correction means that changes an optical characteristic of the target light flux, the optometry controller comprising:
an operation means for operating at least one of the visual target presenting means and the correction means;
a control means that controls at least one of the visual target presenting means and the correction means in response to an operation with respect to the operation means;
an acquisition means that acquires a data log indicating a state of the optometry system in a case where the control means controls at least one of the visual target presenting means and the correction means; and
an output means that outputs the data log.

16. An optometry program used in an optometry system for subjectively measuring an optical characteristic of a subject eye, the optometry system including a visual target presenting means that emits a target light flux toward the subject eye, a correction means that changes an optical characteristic of the target light flux, an operation means for operating at least one of the visual target presenting means and the correction means, and a control unit that controls an action of the optometry system, the optometry program being executed by the control unit to cause the optometry system to perform:
a control step of controlling at least one of the visual target presenting means and the correction means in response to an operation with respect to the operation means;
an acquisition step of acquiring a data log indicating a state of the optometry system in a case where the control means controls at least one of the visual target presenting means and the correction means; and
an output step of outputting the data log.

17. An optometry program used in an optometry system for subjectively measuring an optical characteristic of a subject eye, the optometry system including a visual target presenting means that emits a target light flux toward the subject eye, a correction means that changes an optical characteristic of the target light flux, a first operation means for operating at least one of the visual target presenting means and the correction means, a second operation means different from the first operation means, a display control means that causes a display means of the first operation means to display a first instruction screen for transmitting, to the second operation means, a first trigger signal for controlling an action of the second operation means, and a control unit that controls an action of the optometry system, the optometry program being executed by the control unit to cause the optometry system to perform:
a transmission step of transmitting the first trigger signal from the first operation means to the second operation means in response to an operation with respect to the first instruction screen in the first operation means; and
an output step of causing the second operation means to output first instruction information for assisting an action of the optometry system, based on the first trigger signal transmitted from the first operation means.
